# EUROPÄISCHE PATENTSCHRIFT

(11) **EP 0 699 425 B1**
(45) Veröffentlichungstag und Bekanntmachung des Hinweises auf die Patenterteilung: **17.04.2002**
(21) Anmeldenummer: 94810500.2
(22) Anmeldetag: 01.09.1994
(51) Int. Cl.: A61F 2/34

(54) **Künstliche Hüftgelenkpfanne sowie Verfahren zur Herstellung**
Artificial hip joint acetabular cup as well as manufacturing process
Coque acétabulaire artificielle pour l'articulation de la hanche ainsi que procédé de fabrication

(43) Veröffentlichungstag der Anmeldung: 06.03.1996
(73) Patentinhaber: Sulzer Orthopädie AG, 6340 Baar (CH)
(72) Erfinder: Roland, Willi, CH-8413 Neftenbach (CH)
(74) Vertreter: Manitz, Finsterwald & Partner Gbr

(56) Entgegenhaltungen:
- EP-A- 0 053 794
- EP-A- 0 196 946
- EP-A- 0 297 789
- EP-A- 0 420 795
- EP-A- 0 444 381
- EP-A- 0 453 694
- EP-A- 0 578 322
- FR-A- 2 651 996
- FR-A- 2 668 923
- FR-A- 2 680 674

## Beschreibung

Die Erfindung betrifft eine künstliche Hüftgelenkpfanne gemäss dem Oberbegriff von Anspruch 1. Die Erfindung betrifft weiter ein Verfahren zur Herstellung einer erfindungsgemässen, künstlichen Hüftgelenkpfanne.

Ein künstliches Hüftgelenk umfasst eine Gelenkpfanne sowie eine Gelenkkugel. Diese werden aus unterschiedlichen Werkstoffen wie z.B. oxidkeramische Werkstoffe, Kunststoffe oder Metalle gefertigt, um einerseits den zwischen den Gelenkteilen auftretenden Verschleiss zu reduzieren, und andererseits eine gute Befestigung der Hüftgelenkpfanne im Beckenknochen zu gewährleisten. Aus der EP 0 053 794 ist eine Hüftgelenkpfanne aus oxidkeramischem Werkstoff bekannt. Diese vollständig aus oxidkeramischem Werkstoff bestehende Hüftgelenkpfanne weisen den Nachteil auf, dass sie schlecht im Beckenknochen verankerbar ist.

Aus der FR-A- 2 668 923 ist eine Hüftgelenkpfanne bekannt, welche die Merkmale des Oberbegriffs des Anspruchs 1 aufweist. Die Innenschale dieser Hüftgelenkpfanne ist aus einem Kunststoff, insbesondere Polyethylen, hergestellt, was im Hinblick auf den Verschleiss zwischen den Gelenkteilen nachteilig ist.

Aus der EP-A-0 444 381 ist eine Hüftgelenkpfanne bekannt, die eine metallische Aussenschale und eine metallische Innenschale aufweist. Die Innenschale ist mit der Aussenschale mittels eines speziellen Gewindezapfens verbunden, der einen pilzartigen Kopf aufweist, dessen Aussenrand mittels einer Schnappverbindung in eine konisch hinterschnittene Bohrung der Innenschale einschnappt. Dies ist jedoch eine sehr aufwendige Verbindung.

Die Aufgabe der vorliegenden Erfindung liegt in der Beseitigung der bestehenden Nachteile.

Diese Aufgabe wird gelöst gemäss den Merkmalen von Anspruch 1. Die Unteransprüche 2 bis 8 beziehen sich auf weitere vorteilhafte Ausgestaltungen der Erfindung. Die Aufgabe wird weiter gelöst mit einem Verfahren zur Herstellung einer Hüftgelenkpfanne gemäss den Merkmalen von Anspruch 9. Der Unteranspruch 10 bezieht sich auf ein weiteres vorteilhaftes Verfahren zur Herstellung einer Hüftgelenkpfanne.

Die erfindungsgemässe Hüftgelenkpfanne umfasst mindestens zwei Schalen, eine Innenschale die in eine Aussenschale eingefügt ist. Die Aussenschale ist aus einem plastisch verformbaren Werkstoff hergestellt. Die Innenschale ist aus einem metallischen oder keramischen Werkstoff hergestellt. Sie ist insbesondere aus einem oxidkeramischen Werkstoff, insbesondere aus Aluminiumoxid gefertigt, kann aber auch aus einem anderen Werkstoff wie z.B. einer Kobalt-Chrom Legierung gefertigt sein. Die sphärisch ausgestaltete Innenschale weist einen Pol und auf der dem Pol entgegengesetzten Seite eine äquatoriale Öffnung auf. Die Aussenfläche der Innenschale weist zur äquatorialen Öffnung hin eine Teilfläche mit in Richtung der äquatorialen Öffnung abnehmendem Aussenquerschnitt auf. Die Aussenschale tritt mindestens in dieser Teilfläche in Wirkverbindung mit der Innenschale, derart, dass die Innenschale fest von der Aussenschale gehalten ist. Zumindest in dem Bereich, der um die Teilfläche der Innenschale mit dem zur äquatorialen Öffnung hin abnehmenden Aussendurchmesser angeordnet ist, ist die Aussenschale plastisch verformt, um die Wirkverbindung mit der Innenschale zu bilden

In dem Verfahren zur Herstellung einer erfindungsgemässen Hüftgelenkpfanne wird in einem ersten verfahrensschritt die sphärisch ausgebildete Innnenschale in die sphärisch ausgebildete Aussenschale eingeführt. In einem zweiten Verfahrensschritt wird die Aussenschale zumindest in dem Bereich, der über der besagten Teilfläche der Innenschale liegt, plastisch verformt, derart dass sich zumindest zwischen der besagten Teilfläche der Innenschale und der Aussenschale eine Werkverbindung ergibt, so dass die Innenschale fest durch die Aussenschale gehalten wird. Die plastische Verformung der Aussenschale geschieht z.B. durch bördeln, indem mit einem entsprechenden Werkzeug eine Kraft auf die Aussenschale ausgeübt wird, derart, dass sich die Aussenschale plastisch verformt.

In einer besonders vorteilhaften Ausführungsform liegt die Innenschale zumindest im Bereich der besagten Teilfläche sowie am Pol auf der Innenfläche der Aussenschale auf, so dass die Innenschale festgehalten wird. In einer weiteren vorteilhaften Ausgestaltung der Erfindung liegt die gesamte Aussenfläche der Innenschale auf der Innenfläche der Aussenschale auf.

Ein Vorteil der vorliegenden Erfindung ist darin zu sehen, dass als Innenschale ein Körper aus oxidkeramischem Werkstoff verwendet werden kann, der sich mit einer Aussenschale aus unterschiedlichen Werkstoffen, wie z.B. Titan, Stahl oder Kunststoff zu einer Hüftgelenkpfanne zusammenstellen lässt, wobei die Ausgestaltung der Aussenschale sowie der Werkstoff derart wählbar ist, dass die Aussenschale bezüglich einer Verbindung mit dem Beckenknochen besonders vorteilhafte Eigenschaften aufweist.

Ein weiterer Vorteil der erfindungsgemässen Hüftgelenkpfanne ist darin zu sehen, dass die Innenschale auch aus einem Werkstoff wie eine Kobalt-Chrom Legierung bestehen kann. Auf die Aussenfläche der Aussenschale kann eine weitere Schale angeordnet und damit verbunden werden, z.B. durch Schweissen oder plastische Verformung wie durch bördeln. Somit lässt sich eine drei oder auch mehrschichtige Hüftgelenkpfanne erstellen, die als Innenschale z.B. aus einem oxidkeramischen Werkstoff gefertigt ist, die als Aussenschale eine Titanschale aufweist, und auf deren Aussenschale eine weitere Schale, oder eine weitere Fläche aus Draht, z.B. aus Titandraht aufgebracht ist. Eine solche Hüftgelenkpfanne weist den Vorteil auf, dass die Innenschale geringe Verschleisseigenschaften aufweist, und dass die mit dem Beckenknochen in Berührung kommende Schicht vorteilhafte Eigenschaften bezüglich dem Einwachsen des Beckenknochen aufweist.

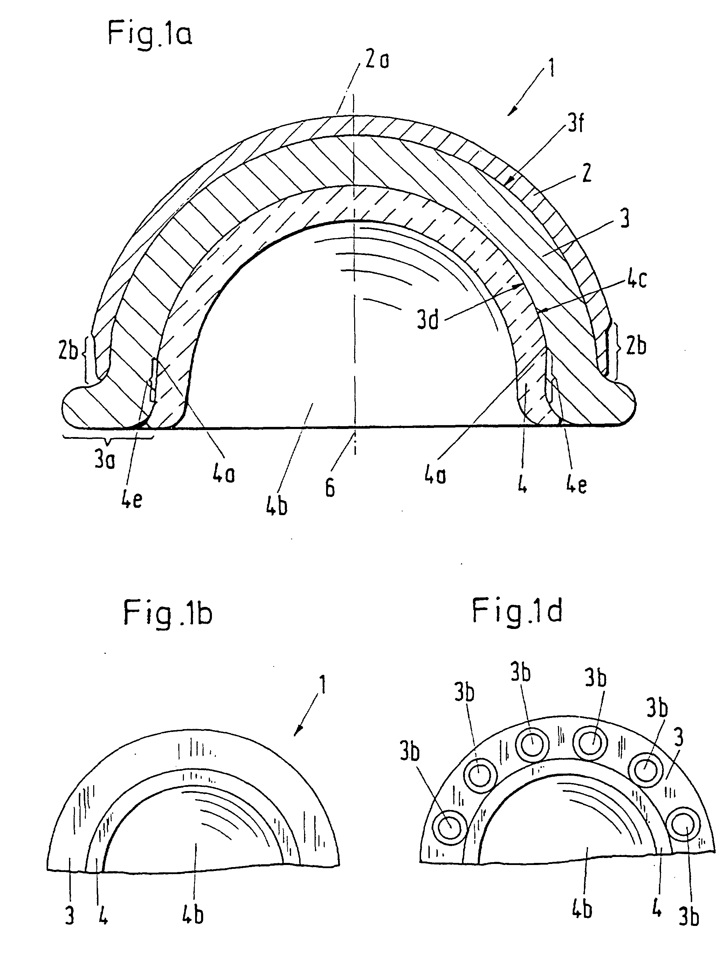

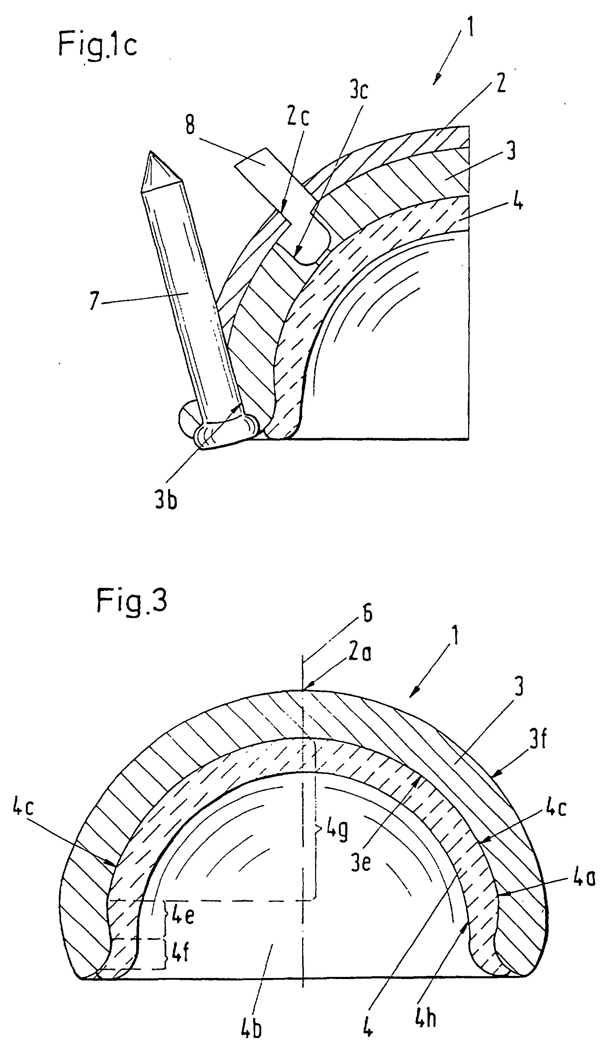

Weitere Einzelheiten und Vorteile der Erfindung ergeben sich aus der Beschreibung von beispielsweisen Ausführungsformen der Erfindung anhand der Figuren 1 a bis 3.

Es zeigen:
- Fig. 1 a: ein Querschnitt durch eine Hüftgelenkpfanne;
- Fig. 1 b: eine Untenansicht einer erfindungsgemässen Hüftgelenkpfanne;
- Fig. 1 c: eine Hüftgelenkpfanne mit angeordneten Befestigungsmitteln;
- Fig. 1 d: eine Unteransicht einer Hüftgelenkpfanne gemäss Fig. 1 c;
- Fig. 2 a: ein Schnitt durch eine weitere Ausführungsform einer Hüftgelenkpfanne;

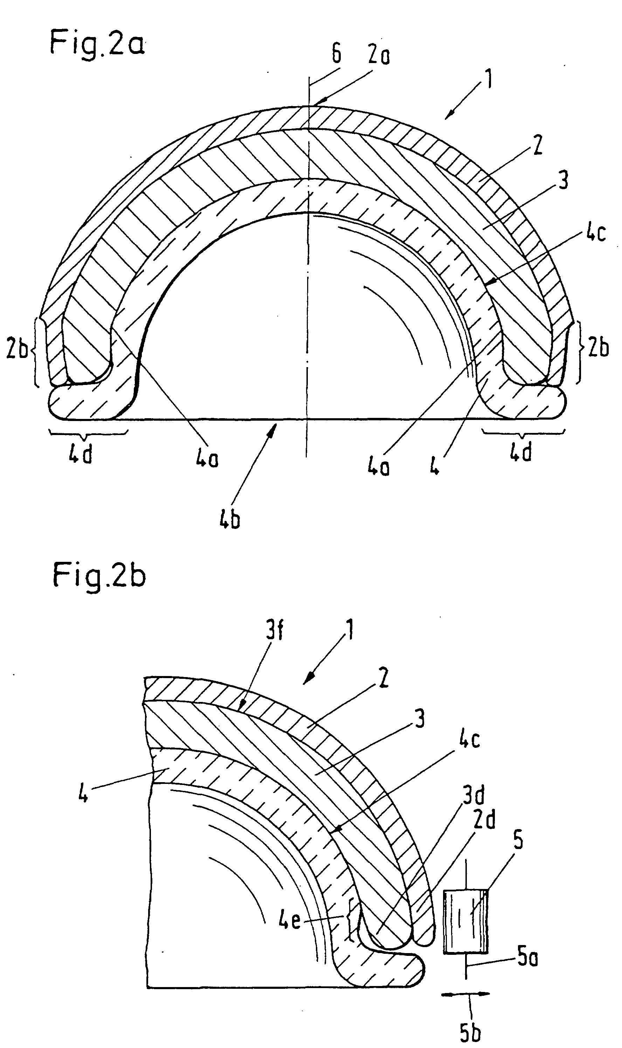

- Fig. 2 b: eine Detailansicht zur Herstellung einer Hüftgelenkpfanne, vor dem plastischen Verformen der Aussenschale;
- Fig. 3: ein Schnitt durch ein weiteres Ausführungsbeispiel einer Hüftgelenkpfanne.

Fig. 3 zeigt eine erfindungsgemässe Hüftgelenkpfanne, mit einer sphärisch ausgebildeten Aussenschale 3, und einer in die Aussenschale 3 eingefügten, sphärisch ausgebildeten Innenschale 4. Die Innenschale 4 kann beispielsweise aus einem oxidkeramischen Werkstoff, insbesondere aus Aluminiumoxid bestehen. Die Aussenschale 4 kann z.B. aus Titan bestehen. Die Hüftgelenkpfanne 1 weist einen Pol 2a, auf deren entgegengesetzten Seite eine kreisförmige, äquatoriale Oeffnung 4b, sowie eine durch den Pol 2a verlaufende Achse 6 auf. Die Innenfläche 4h der Innenschale 4 ist kugelförmig ausgestaltet, zur Aufnahme einer Gelenkkugel. Die Aussenfläche 4c der Innenschale 4 ist derart ausgestaltet, dass sie vom Pol 2a zur äquatorialen Öffnung 4b hin eine erste Teilfläche 4c mit zunehmendem Aussenquerschnitt aufweist, bis zu einer Stelle 4a mit grösstem Aussendurchmesser, dass an die Teilfläche 4c eine weitere Teilfläche 4e anschliesst mit einer zur äquatorialen Öffnung 4b hin abnehmenden Aussenquerschnitt, die zur äquatorialen Öffnung 4b hin in eine weitere Teilfläche 4f mit zunehmendem Aussenquerschnitt übergeht. Die Aussenschale 3 wurde zumindest im Bereich der Teilfläche 4e plastisch verformt, so dass sich zwischen der Aussenschale 3 und der Innenschale 4 mindestens über die Teilfläche 4e eine Werkverbindung ergibt. Für einen sicheren Halt der Innenschale 4 durch die Aussenschale 3 sind mindestens 3 über den Umfang verteilte Werkverbindungen erforderlich.

Es kann sich jedoch auch als vorteilhaft erweisen, dass die Aussenschale 3 über der gesamten Teilfläche 4e eine Werkverbindung zur Innenschale 4 aufweist. Im vorliegenden Ausführungsbeispiel sind die Teilflächen 4e und 4f derart ausgestaltet, wobei die Aussenschale 3 auf beide Teilflächen 4e und 4f eine Werkverbindung ausübt, dass eine Bewegung der Innenschale 4 in Richtung der Achse 6 verhindert wird. Es kann sich als vorteilhaft erweisen, dass die gesamte Aussenfläche 4c der Innenschale 4 im Kontakt steht mit der Innenfläche 3e der Aussenschale 3. Es kann sich jedoch auch als vorteilhaft erweisen, dass die Werkverbindung zwischen der Innenschale 4 und der Aussenschale 3 nur im Bereich der Teilflächen 4e und 4f vorhanden ist, und die übrigen Flächen zwischen der Innenschale und der Aussenschale durch einen Spalt voneinander beabstandet sind.

Fig. 1a zeigt einen Schnitt durch ein weiteres Ausführungsbeispiel einer erfindungsgemässen Hüftgelenkpfanne 1. Auf der Aussenfläche 3f der Aussenschale 3 ist eine weitere Schale 2 angeordnet. Diese weitere Schale 2 kann z.B. aus einem Drahtgeflecht aus Titandraht bestehen, was den bekannten Vorteil aufweist, dass das Knochengewebe gut mit einer derartigen Schicht verwächst. In einer besonders vorteilhaften Ausgestaltung besteht die Innenschale 4 aus einem oxidkeramischen Werkstoff, die Aussenschale 3 aus Titan, und die weitere Schale 2 aus einem Drahtgeflecht aus Titan.

Fig. 1 b zeigt eine Untenansicht der Hüftgelenkpfanne gemäss Fig. 1a, aus der die äquatoriale Öffnung 4b, die Innenschale 4, sowie die Aussenschale 3 ersichtlich ist, die am äquatorialen Rand ein flanschförmiges Teil 3a aufweist. Eine Hüftgelenkpfanne 1, wie sie in Fig. 1a dargestellt ist, eignet sich für eine Verankerung im Beckenknochen ohne Knochenzement. Dabei wird die Aussenschale 3 bzw. die weitere Schale 2 ohne Knochenzement nach dem bekannten Prinzip einer "Press-Fit" oder einer "Snapp-Fit" Pfanne in den Beckenknochen eingeführt und befestigt ist.

Fig. 1c zeigt eine erfindungsgemässe Hüftgelenkpfanne 1 mit Verankerungskörpern 7, 8. Die Hüftgelenkpfanne 1 lässt sich über eine Knochenschraube 7 oder einen Nagel 7 im Beckenknochen befestigen, der durch eine Ausnehmung 3b im flanschförmigen Teil 3a der Aussenschale 3 verläuft.

Weiter können in der Aussenschale 3 Ausnehmungen 3c und bei vorhandener weiteren Schale 2 auch Ausnehmungen 2c vorgesehen sein, derart, dass Verankerungkörper 8, z.B. feste werkmontierte Elemente, oder auch während der Operation montierbare Elemente eingesetzt und befestigt werden können.

Fig. 1d zeigt eine Untenansicht einer erfindungsgemässen Hüftgelenkpfanne gemäss Fig. 1c aus der die kreisförmige, äquatoriale Öffnung 4b, die Innenschale 4, die Aussenschale 3, sowie die Ausnehmungen 3b zur Aufnahme eines Befestigungsmittels 7 ersichtlich sind.

Fig. 2a zeigt ein weiteres Ausführungsbeispiel einer erfindungsgemässen Hüftgelenkpfanne 1, die am äquatorialen Rand ein flanschförmiges Teil 4d aufweist, dass radial zur Achse 6 verläuft. Ein derartiges, flanschförmiges Teil 4d weist den Vorteil auf, dass, wenn die Innenschale 4 aus einem oxidkeramischen Werkstoff gefertigt ist, dass flanschförmige Teil 4d das Einführen der Gelenkkugel leitet. Bekannterweise sind insbesondere oxidkeramische Gelenkkugeln beim Einführen in die Hüftgelenkpfanne sehr verletzungsempfindlich. Im vorliegenden Ausführungsbeispiel wird eine Gelenkkugel auf das flanschförmige Teil 4d aufgesetzt und gleitet nun auf der oxidkeramischen Schicht in den Innenraum der Innenschale 4, so dass eine Verletzung der oxidkeramischen Gelenkkugeln durch vorstehende, metallische Teile ausgeschlossen ist.

In Fig. 2b ist das Verfahren zur Herstellung einer erfindungsgemässen Hüftgelenkpfanne 1 dargestellt. Die sphärisch ausgestaltete Innenschale 4 ist in die sphärisch ausgestaltete Aussenschale 3 eingeführt. Auf der Aussenfläche 3f der Aussenschale 3 ist eine weitere Schale 2 oder Schicht 2d angeordnet. Nach dem Ineinanderführen der Innenschale 4 und der Aussenschale 3 wird die Aussenschale 3 zumindest im Bereich der Teilfläche 4e durch eine Vorrichtung 5 plastisch verformt, derart dass das freiliegende Teil 3d der Aussenschale 3 durch die in radialer Wirkung wirkenden Kräfte plastisch verformt wird und auf die Aussenfläche 4c der Innenschale 4 aufliegt. Im vorliegenden Ausführungsbeispiel wird die plastische Verformung durch bördeln erzielt, indem ein zylinderförmiger, um eine Rotationsachse 5a drehbarer Körper in Bewegungsrichtung 5b zur Hüftgelenkpfanne 1 hin bewegt wird, dabei auf die weitere Schicht 2d wirkt und indirekt somit auch auf das freiliegende Teil 3d der Aussenschale 3. Dies bewirkt ein plastisches Verformen der weiteren Schale 2 sowie der Aussenschale 3, wie dies z.B. in der Fig. 2a durch den gebördelten Bereich 2b der weiteren Schale 2 dargestellt ist. Dies bewirkt einen weiteren Halt der weiteren Schale 2 an der Aussenschale 3, und es lässt sich ein definierter Übergang zwischen der Innenschale 4 und der weiteren Schale 2 oder Schicht 2d erzeugen, so dass die beiden Schalen 4 und 2 z.B. absatzlos ineinander übergehen.

Es ist eine Vielzahl von Verfahren bekannt, die ein plastisches Verformen von Körpern erlauben, so dass das in Fig. 2b gezeigt Ausführungsbeispiel mit einem Verfahren durch bördeln nur als eines aus einer Vielzahl von Verfahren zu betrachten ist, die sich eignen um eine Aussenschale 3 durch plastisches Verformen mit einer Innenschale 4 zu verbinden.

## Patentansprüche

1. Künstliche Hüftgelenkpfanne (1) mit einem Pol (2a), sowie einer äquatorialen Öffnung (4b),
- wobei die Hüftgelenkpfanne (1) mindestens je eine sphärisch ausgebildete Aussenschale (3) und Innenschale (4) umfasst,
- wobei die Innenschale (4) in die Aussenschale (3) eingefügt ist,
- wobei die Aussenfläche (4c) der Innenschale (4) eine Teilfläche (4e) mit einem zur äquatorialen Öffnung (4b) hin abnehmendem Aussenquerschnitt aufweist,
- und wobei die Aussenschale (3) mindestens zur Teilfläche (4e) eine Wirkverbindung aufweist,
um die Innenschale (4) in der Aussenschale (3) zu halten, **dadurch gekennzeichnet,**
**dass** die Innenschale (4) aus einem metallischen oder keramischen Werkstoff hergestellt ist und die Aussenschale aus einem plastisch verformbaren Werkstoff hergestellt ist,
und **dass** die Aussenschale (3) zumindest in dem Bereich, der um die Teifläche (4e) der Innenschale (4) mit dem zur äquatorialen Öffnung (4b) hin abnehmenden Aussendurchmesser angeordnet ist, plastisch verformt ist, um die Wirkverbindung mit der Innenschale (4) zu bilden.

2. Hüftgelenkpfanne (1) nach Anspruch 1, **dadurch gekennzeichnet, dass** die Innenschale (4) an der äquatorialen Öffnung (4b) ein flanschförmiges Teil (4d) aufweist, das über die Aussenschale (3) vorsteht.

3. Hüftgelenkpfanne (1) nach einem der Ansprüche 1 oder 2, **dadurch gekennzeichnet, dass** die Innenschale (4) aus einer Kobalt-Chrom Legierung besteht.

4. Hüftgelenkpfanne (1) nach einem der Ansprüche 1 bis 3, **dadurch gekennzeichnet, dass** die Aussenschale (3) aus Titan oder Stahl besteht.

5. Hüftgelenkpfanne (1) nach einem der Ansprüche 1 bis 4, **dadurch gekennzeichnet, dass** eine weitere Schale (2) auf der Aussenfläche (3f) der Aussenschale (4) aufliegt und mit dieser verbunden ist.

6. Hüftgelenkpfanne (1) nach Anspruch 5, **dadurch gekennzeichnet, dass** die weitere Schale (2) aus Titan oder Stahl besteht und insbesondere als ein Drahtgeflecht ausgeführt ist.

7. Hüftgelenkpfanne (1) nach einem der Ansprüche 1 bis 6, **dadurch gekennzeichnet, dass** die Innenschale (4) und/oder die Aussenschale (3) Durchbrechungen (3b) aufweisen, zur Aufnahme von Befestigungsmitteln wie Knochenschrauben (7).

8. Hüftgelenkpfanne (1) nach einem der Ansprüche 1 bis 7, **dadurch gekennzeichnet, dass** die Aussenschale (3) an deren Aussenfläche (3f) Ausnehmungen (3c) aufweist, zur Aufnahme von Verankerungskörpern (8).

9. Verfahren zur Herstellung einer Hüftgelenkpfanne (1) gemäss Anspruch 1, bei welchem Verfahren zunächst die metallische oder keramische Innenschale (4) in die plastisch verformbare Aussenschale eingesetzt wird und dann die Aussenschale (3) zumindest in dem Bereich, welcher um die Teilfläche (4e) mit dem zur äquatorialen Öffnung hin abnehmenden Aussendurchmesser angeordnet ist, plastisch verformt wird, um zwischen der Aussenschale (3) und der Teilfläche (4e) eine Wirkverbindung zu erzeugen, sodass die Innenschale (4) fest in der Aussenschale (3) gehalten wird.

10. Verfahren nach Anspruch 9, **dadurch gekennzeichnet, dass** eine weitere Schale (2) auf die Aussenschale (3) aufgebracht wird, und dass die weitere Schale (2) plastisch verformt wird, derart, dass die weitere Schale (2) fest mit der Aussenschale (3) verbunden wird.

## Claims

1. Artificial acetabulum (1) with a pole (2a), as well as an equatorial opening (4b),
- with the acetabulum (1) comprising at least one spherically formed outer shell (3) and at least one inner shell (4),
- with the inner shell (4) being inserted into the outer shell (3),
- with the outer surface (4c) of the inner shell (4) having a partial surface (4e) with an outer cross-section decreasing towards the equatorial opening (4b),
- and with the outer shell (3) having an operative connection at least towards the partial surface (4e), in order to retain the inner shell (4) in the outer shell (3), **characterised in that**
the inner shell (4) is manufactured of a metallic or ceramic material and the outer shell is manufactured from a plastically deformable material;
and **in that** the outer shell is plastically deformed, at least in the region which is disposed around the partial surface (4e) of the inner shell (4) with the outer diameter reducing towards the equatorial opening (4b), in order to form the operative connection to the inner shell (4).

2. Acetabulum (1) in accordance with claim 1, **characterised in that** the inner shell (4) has a flange-like part (4d) at the equatorial opening (4b), with the flange-like part (4d) projecting beyond the outer shell (3)

3. Acetabulum (1) in accordance with one of the claims 1 or 2, **characterised in that** the inner shell (4) consists of a cobalt-chromium-alloy.

4. Acetabulum (1) in accordance with one of the claims 1 to 3, **characterised in that** the outer shell (3) consists of titanium or steel.

5. Acetabulum (1) in accordance with one of the claims 1 to 4, **characterised in that** a further shell (2) lies on the outer surface (3f) of the outer shell (4) and is connected to it.

6. Acetabulum (1) in accordance with claim 5, **characterised in that** the further shell (2) consists of titanium or steel, in particular formed as a wire braid.

7. Acetabulum (1) in accordance with one of the claims 1 to 6, **characterised in that** the inner shell (4) and/or the outer shell (3) has apertures (3b) for receiving securing means, such as bone screws (7).

8. Acetabulum (1) in accordance with one of the claims 1 to 7, **characterised in that** the outer shell (3) has apertures (3c) at its surface (3f) for receiving anchoring bodies (8).

9. Method for manufacturing an acetabulum (1) in accordance with claim 1, in which method the metallic or ceramic inner shell is first inserted into the plastically deformable outer shell and then the outer shell (3) is plastically deformed, at least in the region which is disposed around the partial surface (4e), with the outer diameter reducing towards the equatorial opening, in order to generate an operative connection between the outer shell (3) and the partial surface (4e), so that the inner shell (4) is-fixedly held in the outer shell (3).

10. Method in accordance with claim 9, **characterised in that** a further shell (2) is applied to the outer shell (3), and **in that** the further shell (2) is plastically deformed, such that the further shell (2) is fixedly connected to the outer shell (3).

## Revendications

1. Coque acétabulaire artificielle (1) avec un pôle (2a), et avec une ouverture équatoriale (4b),
- où la coque acétabulaire (1) comprend au moins respectivement une coque externe (3) et une coque interne (4) réalisées d'une manière sphérique,
- où la coque interne (4) est insérée dans la coque externe (3),
- où la face externe (4c) de la coque interne (4) présente une face partielle (4e) d'une section transversale extérieure diminuant vers l'ouverture équatoriale (4b)
- et où la coque externe (3) présente au moins vers la face partielle (4e) une liaison active pour retenir la coque interne (4) dans la coque externe (3), **caractérisée en ce que** la coque interne (4) est réalisée en un matériau métallique ou céramique et que la coque externe est réalisée en un matériau déformable plastiquement, et **en ce que** la coque externe (3), au moins dans la zone disposée autour de la face partielle (4e) de la coque interne (4) du diamètre extérieur diminuant vers l'ouverture équatoriale (4b), est déformée plastiquement pour réaliser la liaison active avec la coque interne (4).

2. Coque acétabulaire (1) selon la revendication 1, **caractérisée en ce que** la coque interne (4) présente à l'ouverture équatoriale (4b) une partie en forme de bride (4d) qui fait saillie sur la coque externe (3).

3. Coque acétabulaire (1) selon l'une des revendications 1 à 2, **caractérisée en ce que** la coque interne (4) est constituée d'un alliage de cobalt-chrome.

4. Coque acétabulaire (1) selon l'une des revendications 1 à 3, **caractérisée en ce que** la coque externe (3) est constituée de titane ou d'acier.

5. Coque acétabulaire (1) selon l'une des revendications 1 à 4, **caractérisée en ce qu'**une couche supplémentaire (2) repose sur la face extérieure (3f) de la coque externe (4) et est reliée à celle-ci.

6. Coque acétabulaire (1) selon la revendication 5, **caractérisée en ce que** la coque supplémentaire (2) est constituée de titane ou d'acier et est réalisée notamment comme un tressage en fils.

7. Coque acétabulaire (1) selon l'une des revendications 1 à 6, **caractérisée en ce que** la coque interne (4) et/ou la coque externe (3) présente des perçages (3b) pour la réception de moyens de fixation, comme des vis à os (7).

8. Coque acétabulaire (1) selon l'une des revendications 1 à 7, **caractérisée en ce que** la coque externe (3) présente à sa face extérieure (3f) des évidements (3c), pour la réception de corps d'ancrage (8).

9. Procédé de fabrication d'une coque acétabulaire (1) selon la revendication 1, dans lequel la coque interne métallique ou céramique (4) est d'abord placée dans la coque externe déformable plastiquement et qu'ensuite, la coque externe (3), au moins dans la zone qui est disposée autour de la face partielle (4e) avec le diamètre extérieur diminuant vers l'ouverture équatoriale, est déformée plastiquement pour produire entre la coque externe (3) et la face partielle (4e) une liaison active de façon que la coque interne (4) soit retenue solidement dans la coque externe (3).

10. Procédé selon la revendication 9, **caractérisé en ce qu'**une coque supplémentaire (2) est placée sur la coque externe (3), et **en ce que** la coque supplémentaire (2) est déformée plastiquement de façon que la coque supplémentaire (2) soit reliée solidement à la coque externe (3).
